Europäisches Patentamt

European Patent Office

Office européen des brevets

(11) Veröffentlichungsnummer: **0 271 907 B1**

(19)

(12) **EUROPÄISCHE PATENTSCHRIFT**

(45) Veröffentlichungstag der Patentschrift: **01.04.92**

(51) Int. Cl.⁵: **C12P 19/04**, C08B 37/00

(21) Anmeldenummer: **87118712.6**

(22) Anmeldetag: **17.12.87**

(54) **Hochmolekulare Homopolysaccharide, Verfahren zu ihrer extrazellulären Herstellung und zu ihrer Anwendung, sowie die entsprechenden Pilzstämme.**

(30) Priorität: **19.12.86 DE 3643467**

(43) Veröffentlichungstag der Anmeldung:
**22.06.88 Patentblatt 88/25**

(45) Bekanntmachung des Hinweises auf die Patenterteilung:
**01.04.92 Patentblatt 92/14**

(84) Benannte Vertragsstaaten:
**AT DE FR GB NL**

(56) Entgegenhaltungen:
**FR-A- 2 406 447**

**CHEMICAL ABSTRACTS, Band 99, Nr. 23, Dezember 1983, Seite 615, Zusammenfassung Nr. 193208z, Columbus, Ohio, US; & JP-A-58 138 392 (JAPAN SYNTHETIC RUBBER CO. LTD) 17-08-1983**

**CARBOHYDRATE RESEARCH, Band 117, Nr. 1/2, Juni 1983, Seiten 201-213, Elsevier Science Publishers B.V., Amsterdam, NL; C. HARA et al.: "A branched (1-3)-beta-D-glucan from a sodium carbonate extract of Dictyophora indusiata fisch"**

(73) Patentinhaber: **WINTERSHALL AKTIENGESELL-SCHAFT**
**Postfach 10 40 20 Friedrich-Ebert-Strasse 160**
**W-3500 Kassel(DE)**

(72) Erfinder: **Lindoerfer, Walter, Dr.**
**Christian-Beyer-Strasse 16**
**W-3500 Kassel(DE)**
Erfinder: **Sewe, Kai-Udo, Dr.**
**Zuckmayerstrasse 6**
**W-2847 Barnstorf(DE)**
Erfinder: **Wagner, Fritz, Prof. Dr.**
**Hohe Wiese 2**
**W-3301 Stoeckheim(DE)**
Erfinder: **Muenzer, Sylvia**
**Schoeppenstedter Strasse 40**
**W-3300 Braunschweig(DE)**
Erfinder: **Nachtwey, Sabine**
**Rosenstrasse 8**
**W-3303 Vechelde(DE)**
Erfinder: **Rapp, Peter, Dr.**
**Schlegelstrasse 11**
**W-3300 Braunschweig(DE)**
Erfinder: **Rau, Udo, Dr.**
**Buchenweg 8 b**
**W-3305 Dettum(DE)**

EP 0 271 907 B1

PATENT ABSTRACTS OF JAPAN, Band 7, Nr. 252 (C-194)[1397], 9. November 1983, Seite 73 C 194; & JP-A-58 138 392 (NIPPON GOSEI GOMU K.K.) 17-08-1983

PATENT ABSTRACTS OF JAPAN, Band 10, Nr. 36 (C-328)[2093], 13. Februar 1986; & JP-A-60 188 402 (HAYASHIBARA SEIBUTSU KAGAKU KENKYUSHO K.K.) 25-09-1985

Erfinder: **Stephan, Doerte, Dr.**
**Amalienstrasse 2-3**
**W-3300 Braunschweig(DE)**

(74) Vertreter: **Höller, Klaus, Dr. et al**
**BASF Aktiengesellschaft Carl-Bosch-Strasse 38**
**W-6700 Ludwigshafen(DE)**

**Beschreibung**

Wasserlösliche Biopolymere haben in der Technik für verschiedene Anwendungsgebiete Verwendung gefunden. Die amerikanische Patentschrift 3 301 848 (which is hereby enclosed by reference) beschreibt wasserlösliche, nichtionische Polysaccharide definierter Struktur als Glucane. Diese werden mikrobiell durch Züchtung des Mikroorganismus Sclerotium glucanicum n.sp., NRRL 3006, als Metaboliten erzeugt. Sie sind jedoch relativ niedermolekular. Das Gleiche gilt für die in GB 2 178 437 beschriebenen Polysaccharide.

W.L. Griffith und A.L. Compere beschreiben in Dev. Ind.Microbiol. 19, 609-617, (1977). die mikrobielle Erzeugung von hoch-viskosen Glucanen durch Kultivierung des Mikroorganismus Sclerotium rolfsii, ATCC 15206, in einem Nährmedium mit Gehalt an anorganischen Salzen, Glucose und einer geringen Menge an Hefeextrakt mit einer Züchtungsdauer von 2,5 Tagen. Die erzeugten Glucane zeigen eine Abnahme der Löslichkeit in Wasser bei pH-Werten von 1,5 bis 4.

Gegenstand der Erfindung ist ein Verfahren zur extrazellulären Herstellung von Homopolysacchariden (PS) mit hoher spezifischer Viskosität, die Pilzstämme, die diese PS produzieren, die PS selbst und ihre Verwendung.

Es ist die Aufgabe der Erfindung, nichtionische Homopolysaccharide mit hoher spezifischer Viskosität, hoher Temperatur- und Salz-Stabilität und mit Langzeit-Stabilität herzustellen. Mit "Stabilität" ist gemeint, daß sich das Molekulargewicht und damit die Eigenschaften unter dem (ggf. gleichzeitigen) Einfluß von erhöhter Temperatur, Salzen und Luftsauerstoff nicht wesentlich vernändert.

Anspruch 1 betrifft die Pilzstämme DSM 3887, DSM 3888, DSM 3890, DSM 3891, DSM 3892, die bei der Deutschen Sammlung für Mikroorganismen (DSM), Göttingen, hinterlegt sind.

Das Verfahren der Erfindung ist im kennzeichnenden Teil des Patentanspruches 2 definiert.

Die Aufgabe der Erfindung wird dadurch gelöst, daß Mirkoorganismen in der Form der in den Ansprüchen 1 und 2 genannten Pilzstämme in einem Nährmedium unter Belüftung und Bewegung bei einer Temperatur von etwa 15°C bis 40°C gezüchtet werden, danach für 2 bis 30, vorzugsweise 5 bis 15 min. auf 70 bis 90, vorzugsweise 75 bis 85°C erhitzt werden, falls die Pilzstämme ausgereift sind, danach die Kulturlösung von der Zellmasse abgetrennt und das gebildete Homopolysaccharid mit praktisch nur Glucose als Monomergrundkomponente mit einem Reinheitsgrad im Bereich von 0,03 bis 0,1 Gew.% Restprotein daraus isoliert wird.

Als Nährmedium werden den Pilzstämmen die üblichen, für sie verwertbaren Stickstoff- und Kohlenstoffquellen sowie die zum Wachstum notwendigen anorganischen Ionen zur Verfügung gestellt, und zwar zweckmäßig in einer solchen Konzentration, daß beim Abbruch der Kultivierung eine Restkonzentration an der Kohlenstoffquelle in einer Größenordnung von 0,1 Gew.%, bezogen auf die gesamte Lösung, besteht. Die erzeugten nichtionischen PS haben eine Vikosität im Bereich von 50 bis 190 m Pa•s bei einer Scherrate $D = 0,3/s^{-1}$ bei 40°C, bestimmt mit 0,3 g PS, gelöst in 1000 ml Wasser hoher Salinität (mit bis 100 g/l Alkali-Tonen und bis 30 g/l Erdalkali-Ionen). Diese Viskosität bleibt bei einer Temperatur von 60°C an der Luft 1 Jahr und länger praktisch konstant. Unter Sauerstoffausschluß gilt dies sogar für Temperaturen bis 90°C. Die Viskosität der erfindungsgemäß erzeugten nichtionischen PS wird bei einer Konzentration von 0,1 bis 1 g/l im Temperaturbereich von etwa 20 bis 60°C und im Scherbereich von etwa $D = 3,0 \, s^{-1}$ und größer nur wenig beeinflußt, so daß sie für praktische Zwecke als konstant angesehen werden kann. Auch nach einstündigem Erhitzen einer 0,3 g/l enthaltenden Lösung der erfindungsgemäß erhältlichen PS im Autoklaven bei neutralem pH-Wert auf 120°C bei 1 bar Überdruck ist die Viskosität nach Abkühlen auf 20°C und Ergänzen des Verdunstungswassers im Scherbereich von $D = 0,1$ bis $300 \, s^{-1}$ praktisch nicht beeinflußt. Die PS bestehen aus einer linearen Kette 1,3-verknüpfter β-D-Glucopyranose mit unterschiedlichem Substitutionsgrad 1,6-gebundener β-D-Glucopyranosyl-Gruppen.

Die Pilzstämme werden für den Einsatz zweckmäßig an einer Trägersubstanz, vorzugsweise einem Polyurethanschaum, in üblicher Weise immobilisiert und mit diesem Immobilisat wird die Bildung der PS semikontinuierlich oder vollkontinuierlich durchgeführt.

Die erfindungsgemäß erhältlichen PS eignen sich hervorragend zum Einsatz als Dispergier- oder Emulgiermittel (allein oder in Kombination mit anionischen und/oder nichtionischen Tensiden), als strukturviskose Verdickungsmittel, als Wasserrückhaltemittel sowie für die Verbesserung der Haftung von Stoffen an festen Oberflächen. Bei der Verwendung als Verdickungsmittel ist insbesondere an den Einsatz bei Bohrspülungen, bei sekundärem und tertiärem Fluten von Erdöllagerstätten und generell zur Herabsetzung von Reibungswiderständen in strömenden, insbesondere in turbulenten wäßrigen Flüssigkeiten zwecks Herabsetzung des Druckverlustes gedacht. Als Dispergiermittel kommen beispielsweise Anwendungen bei landwirtschaftlichen Chemikalien wie Fungiziden, Pestiziden sowie im Nahrungsmittel- und Futtermittelbereich in Betracht.

Die Filtrationseigenschaften der erfindungsgemäß erzeugten PS als Ausdruck der Injizierbarkeit in poröse Medien wie Erdölträgergestein sind hervorragend: Sie liegen bei einer Probe von 0,3 g PS/l Wasser mit hoher Salinität mit bis 100 g/l Alkali-Ionen und bis 30 g/l Erdalkali-Ionen bei einer Druckfiltration mit einem bar Überdruck über ein 3 $\mu$m Membranfilter mit 200 ml Lösung 5 sec. geschert im Bereich von 0,5 bis 3,0 min. und ungeschert im Bereich von 0,8 bis 10 min., oder über ein 1,2 $\mu$m Membranfilter 5 sec. geschert im Bereich von 1,6 bis 18 min. und ungeschert im Bereich von 4,0 bis 14 min..

Die Züchtung der Suspensionskultur wird auf etwa 40 bis 180, vorzugsweise 50 bis 80 Stunden eingestellt.

Das erfindungsgemäße Verfahren liefert die PS unmittelbar in hochreiner Form. Der Restproteingehalt liegt im Bereich von etwa 0,03 bis 0,1 Gew.%.

"Wasserlöslich" im Sinne der Erfindung heißt, daß mindestens 1, vorzugsweise mindestens 20 g PS im Liter Wasser von 20°C löslich sind. Die erfindungsgemäßen PS sind trotz ihres hohen Molekulargewichtes wasserlöslich, weil sie in hohem Grade durch Seitenketten substituiert sind. Sie unterscheiden sich von den in US 3 301 848 beschriebenen Polysacchariden u.a. durch ihr wesentlich höheres Molekulargewicht. Hauptsächlich darauf beruhen ihre überlegenen Eigenschaften.

Die Charakterisierung der bevorzugt eingesetzten Mikroorganismen in Form von Pilzstämmen ist aus der folgenden Tabelle ersichtlich.

Charakterisierung der Pilzstämme

| Pilzstämme: | DSM 3887+ | DSM 3888 | DSM 3889 | DSM 3890 | DSM 3891 | DSM 3892 |
|---|---|---|---|---|---|---|
| Morphologie | | | | | | |
| Luftmyzel | + | + | ++ | + | + | ++ |
| Farbe | schwarz | weiß | weiß | weiß | weiß | weiß |
| Farbstoffaus- scheidung | schwarz | - | - | - | schwarz | - |
| Dauerorgane | Bulbillen | - | Sclerotien | Sclerotien | Sclerotien | Sclerotien |
| Fruchtkörper | - | Basidien | - | Apothezien | Apothezien | Ascus |
| Wachstum in Submerskultur | | | | | | |
| aerob | + | + | + | + | + | + |
| Temp. Opt. $^{0}$C | 27 | 27 | 27 | 25 | 25 | 25 |
| Anfangs pH | 4.5 | 5.3 | 3.5 | 5.7 | 5.6 | 5.7 |
| End pH | 3.0 | 4.6 | 2.5 | 3.5 | 3.5 | 3.5 |
| Pelletbildung | ± | ++ | + | + | + | + |
| Polysaccharid | AW 110 | AW 114 | AW 106 | AW 115 | AW 116 | AW 117 |

+) = offizielle Hinterlegungsnummer bei der Deutschen Sammlung von Mikroorganismen (DMS), Grisebachstr. 8, D-3400 Göttingen

-) = Eigenschaft nicht vorhanden

± = Eigenschaft schwach ausgeprägt

+ = Eigesnchaft deutlich vorhanden

++ = Eigenschaft stark ausgeprägt

EP 0 271 907 B1

Fortsetzung

| Pilzstämme: | DSM 3887 | DSM 3888 | DSM 3889 | DSM 3890 | DSM 3891 | DSM 3892 |
|---|---|---|---|---|---|---|
| **Enzymatische Aktivitäten** | | | | | | |
| 1,3-$\beta$-Glucanase | + | + | + | + | + | + |
| 1,6-$\beta$-Glucanase | + | + | + | + | + | + |
| Chitinase | + | + | + | + | + | + |
| 1,4-$\alpha$-Glucanase | − | + | + | + | + | + |
| 1,4-$\beta$-Glucanase | − | + | + | − | − | − |
| Xylanase | − | + | − | + | − | − |
| | | | | | | |
| **Verwertbare N-Quellen** | | | | | | |
| Ammonium-ionen | − | + | + | + | + | + |
| Nitrat-ionen | + | − | + | − | − | − |
| Harnstoff | − | + | + | + | − | + |
| Asparagin | + | + | + | + | + | + |
| Glutamin | + | + | + | + | + | + |
| | | | | | | |
| **Verwertbare C-Quellen** | | | | | | |
| Arabinose | + | − | − | − | + | − |
| Xylose | + | + | + | + | + | + |
| Fructose | + | + | + | + | + | + |
| Glucose | + | + | + | + | + | + |
| Saccharose | + | + | + | + | + | + |
| Maltose | + | + | + | + | + | + |
| Cellobiose | + | + | + | − | − | + |
| Stärke | − | + | + | + | + | + |
| Cellulose | − | + | + | − | − | + |
| Xylan | − | + | − | + | − | − |

+: Eigenschaft vorhanden          −: Eigenschaft nicht vorhanden

EP 0 271 907 B1

| | AW | | | | | |
|---|---|---|---|---|---|---|
| | 106 | 110 | 114 | 115 | 116 | 117 |
| $\eta^0$ [mPas] | 187 | 81 | 188 | 51 | 163 | 144 |
| n | 0.1 | 0.15 | 0.06 | 0.12 | 0. 15 | 0.13 |
| [$\eta$] [ml/g] | 62000 | 91000 | 17000 | - | - | 43000 |
| MG [g/mol] | $22.5 \cdot 10^6$ | $9 \cdot 10^6$ | $8 \cdot 10^6$ | - | - | $17 \cdot 10^6$ |
| PG | 139000 | 55000 | 50000 | - | - | 105000 |

$\eta^0$ = Ruhescherviskosität (zero shear viscosity) einer wäßrigen PS-Lösung mit 0.3 g/l

n = Fließindex (flow behaviour index) Erklärung siehe Veröffentlichung of U. Rau and F. Wagner "Non-Newtonian Flow Behaviour of Colloid-Disperse Glucan Solutions". Biotechnology Letters 9. 95-100 (1987).

[$\eta$] = Staudinger Index (intrinsic viscosity) gibt den Raumbedarf eines Polymerknäules in verdünnter Losung (< =0,5 g/l) an und steht über die Mark-Houwink Gleichung n = K$\cdot$MG$^a$ mit dem MG in direkter Beziehung

MG = Molekulargewicht

PG = Polymerisationsgrad MG/162 162 = D-Glucose - 1 $H_2O$

Das Verfahren der Erfindung wird durch die folgenden Beispiele erläutert.

Beispiel 1

Herstellung eines Homopolysaccharides, Typ AW 106, mit dem Pilzstamm DSM 3889.

Zwei Schrägagarkulturen des Stammes DSM 3889 wurden in je 3 ml 0,9 %iger Kochsalzlösung abgeschwemmt und damit 500 ml einer Nährlösung, enthaltend 1,5 g $NaNO_3$, 0,5 g $K_2HPO_4$ $\cdot$ 3 $H_2O$, 0,25 g $MgSO_4$ $\cdot$ 7 $H_2O$, 0,25 g KCL, 1,0 g Hefeextrakt und 5,0 g Glucose beimpft. Unter Schütteln wurde die Suspensionskultur bei 27°C 60 Stunden bei pH 4,5 gezüchtet. Mit der so hergestellten Vorkultur wurde ein 15 l Bioreaktor, ausgerüstet mit drei sechsblättrigen Scheibenrührern, beimpft, der 10 l eines Kulturmedium folgender Zusammensetzung enthielt:

$NaNO_3$        30 g

$K_2HPO_4$ $\bullet$ 3 $H_2O$        13 g

$MgSO_4$ $\bullet$ 7 $H_2O$        5.0 g

KCl        5.0 g

$ZnSO_4$ $\bullet$ 7 $H_2O$        0.03 g

Citronensäure $\bullet$ $H_2O$        7.0 g

Glucose        300.0 g

Die Züchtung wurde 80 Stunden bei 27°C unter Rühren mit 500 Upm und einer Belüftung von 1.67 Liter steriler Luft pro Liter Nährmedium und Minute bei einem Anfangs-pH-Wert von 3.5 durchgeführt. Während der Kultivierung fiel der pH-Wert auf 2.7, nach Beendigung der Züchtung war der Restglukosegehalt kleiner 0,1 Gew.%.

Die Kultursuspension wurde 30 Minuten auf 80°C erhizt, mit 50 l deionisiertem Wasser verdünnt und bei 15000 g zentrifugiert. Das Polysaccharid AW 106 wurde aus dem überstand mit i-Propanol gefällt (1:1 Volumenverhältnis), abfiltriert, mit 70 %iger wäßriger i-Propanollösung gewaschen, der Niederschlag in 1 l deionisiertem Wasser resolvatisiert und gefriergetrocknet. Es wurden 120 g Polysaccharid AW 106 erhalten. Das Produkt enthielt als Monomerbaustein Glucose und weniger als 0,05 Gew.% Protein. 3.0 g AW 106, gelöst in 1000 ml Lagerstättenwasser (Zusammensetzung g/l: $CaCl_2$ 42,6 $MgCl_2$ 10,5, NaCl 138,0, $Na_2SO_4$ 1.2 und $NaBO_2$ $\bullet$ 4 $H_2O$ 0.4) besaß bei einer Scherrate von 0.3 $s^{-1}$ und 40°C eine Viskosität von 137,7 mPas.

Beispiel 2

Herstellung eines Homopolysaccharides, Typ AW 110, mit dem Pilzstamm DSM 3887.

Vier Schrägagarkulturen des Stammes DSM 3887 wurden in je 3 ml 0,9 %iger Kochsalzlösung abgeschwemmt und damit 1000 ml einer Nährsalzlösung, enthaltend 1.0 g $K_2HPO_4 \bullet 3\,H_2O$, 1.5 g KCl, 1,5 g $NaNO_3$, 0.5 g $MgSO_4 \bullet 7\,H_2O$, 0.5 g Hefeextrakt und 10.0 g Glucose beimpft. Unter Schütteln wurde die Suspensionskultur bei 27° C 54 Stunden bei pH 4,5 gezüchtet. Mit dieser Impfkultur wurde ein 70 1-Bioreaktor, ausgerüstet mit einem Intensor®-System der Fa. Giovanola, Monthey, Schweiz, beimpft, der 50 l eines Nährmediums folgender Zusammensetzung enthielt:

| | |
|---|---|
| $NaNO_3$ | 75 g |
| KCL | 75 g |
| $K_2HPO_4 \bullet 3\,H_2O$ | 50 g |
| $MgSO_4 \bullet 7\,H_2O$ | 25 g |
| $FeSO_4 \bullet 7\,H_2O$ | 2.5 g |
| Xylose | 1500.0 g |

Die Züchtung wurde 96 Stunden bei 27° C unter Rühren mit 1200 Upm und einer Belüftung von 0.5 Liter steriler Luft pro Liter Nährmedium und Minute bei einem Anfangs-pH-Wert von 4.5 durchgeführt. Nach Beendigung der Kultivierung war der pH-Wert auf 3.0 abgefallen und die Xylose nahezu verbraucht.

Das Kulturmedium wurde auf 85° C erhitzt und bei dieser Temperatur über eine Druckfiltration die Zellmasse abgetrennt. Das klare, hochviskose Filtrat wurde, wie in Beispiel 1 beschrieben, mit Isopropanol gefällt und gefriergetrocknet. Es wurden 435 g des Homopolysaccharids, Typ AW 110, gewonnen. Das Produkt enthielt als Monomergrundkomponente ausschließlich Glucose mit einem Restgehalt an Protein von unter 0,03 Gew.%.

Die Elementaranalyse ergab folgende Zusammensetzung:

Kohlenstoff: 44,5 %

Wasserstoff: 6,25 %

Stickstoff: 0,005 %

Es ergaben 0,3 g AW 110 in 1000 ml Lagerstättenwasser der Erdölgewinnung mit der Zusammensetzung gemäß Beispiel 1 bei einer Scherrate von 0,3 $s^{-1}$ und 40° C eine Viskosität von 81.6 mPas.

Beispiel 3

Herstellung eines Homopolysaccharides, Typ AW 114, mit dem Pilzstamm DSM 3889.

Zwei Schrägagarkulturen des Stammes DSM 3888 wurden in je 3 ml 0.9 %iger Kochsalzlösung suspendiert und mit dieser Suspension 500 ml einer Nährsalzlösung, enthaltend 3.0 g Hefeextrakt, 0.75 g $(NH_4)_2HPO_4$, 0.5 g $K_2HPO_4 \bullet 3\,H_2O$, 0.25 g $MgSO_4 \bullet 7\,H_2O$ und 10 g Glucose beimpft. Unter Schütteln wurde die Suspensionskultur bei 27° C 96 Stunden bei pH 5,3 gezüchtet. Die gebildeten Pellets wurden mit einem Ultra-Turrax®-Rührer bei 20.000 Upm 10 s homogenisiert, und mit dieser homogenisierten Impfkultur wurde ein 15 l-Bioreaktor, ausgerüstet mit drei sechsblättrigen Scheibenrührern, beimpft, der 10 l eines Nährmediums folgender Zusammensetzung enthielt:

| | |
|---|---|
| Techn. Hefeextrakt | 30.0 g |
| $K_2HPO_4 \bullet 3\,H_2O$ | 10.0 g |
| $MgSO_4 \bullet 7\,H_2O$ | 5.0 g |
| Xylose | 250.0 g |

Die Züchtung wurde 76 Stunden bei 27° C unter Rühren mit 400 Upm und einer Belüftung von 0.1 Liter steriler Luft pro Liter Nährmedium und Minute bei einem Anfangs-pH-Wert von 5.3 durchgeführt. Nach Beendigung der Kultivierung war der pH-Wert auf 4.6 abgefallen und der Rest-Xylose-Gehalt betrug 0.1 Gew.%.

Der Reaktionsansatz wurde auf 80° C aufgeheizt, mit 10 g Formaldehyd versetzt und durch Scherung mit einem Ultra-Turrax 10 s bei 20.000 Upm das Polysaccharid überwiegend aus den entstandenen Pellets freigesetzt. Danach wurde das Zellmaterial durch eine Druckfiltration abgetrennt und das klare Filtrat durch Ultrafiltration bei einer Ausschlußgrenze von 100.000 Dalton von niedermolekularen Substanzen befreit. Das Ultrafiltrat wurde wie in Beispiel 1 beschrieben mit Isopropanol gefällt und gefriergetrocknet. Es wurden 72 g des Homopolysaccharides AW 114 erhalten. Das Produkt enthielt als Monomergrundkomponenten ausschließlich Glucose mit einem Restgehalt an Protein von unter 0,05 Gew.%.

Die Elementaranalyse ergab folgende Zusammansetzung:

Kohlenstoff:      44,42 %
Wasserstoff:       6,19 %
Stickstoff:        0,01 %

0,3 g AW 114, gelöst in 1000 ml Lagerstättenwasser der Zusammensetzung wie in Beispiel 1, ergab bei einer Scherrate von 0,3 s⁻¹ und 40°C eine Viskosität von 188,7 mPas.

Beispiel 4

Herstellung eines Homopolysaccharides, Typ AW 115, mit dem Pilzstamm DSM 3890.

Eine Schrägagarkulturen des Stammes DSM 3890 wurde in 3 ml 0,9 %iger Kochsalzlösung abgeschwemmt und damit 100 ml einer Nährsalzlösung, enthaltend 0.1 g Hefeextrakt, 0.1 g $K_2HPO_4$ • 0.5 g $MgSO_4$ • 7 $H_2O$ und 1.0 g Glucose beimpft. Unter Schütteln auf einer Rotationsschüttelmaschine bei 110 Upm wurde die Suspensionskultur bei 25°C 60 Stunden bei pH 5,7 gezüchtet. Mit dieser Vorkultur wurden zwei 2 l-Erlenmeyerkolben beimpft, die je 500 l Nährmedien enthielten mit folgender Zusammensetzung:

$K_2HPO_4$ • 3 $H_2O$          0.5 g
$MgSO_4$ • 7 $H_2O$          0.25 g
Hefeextrakt          0.5 g
Glucose          12.0 g

Nach Inkubation bei 25°C auf einer Rotationsschüttelmaschine bei 110 Upm und einem pH-Wert von 5.7 war die vorgegebene Glucose nach 142 Stunden verbraucht und ein pH-Wert von 3.5 erreicht. Die beiden Ansätze wurden vereinigt, mit 5 l deionisiertem Wasser verdünnt und die Zellmasse durch Zentrifugation abgetrennt. Der klare Überstand wurde durch Ultrafiltration bei einer Ausschlußgrenze von 100.000 Dalton auf einen Polysaccharidgehalt von 4.3 g/l aufkonzentriert bei gleichzeitiger Abtrennung der niedermolekularen Komponenten. Das Konzentrat wurde mit Ethanol (Volumenverhältnis 1:1) versetzt, das ausgefallene Polysaccharid AW 115 abzentrifugiert, mit 200 ml 70 %igem Ethanol gewaschen und gefriergetrocknet. Es wurden 5.05 g Polysaccharid AW 115 erhalten. Dieses Produkt enthielt als Monomerbaustein ausschließlich Glucose und enthielt als Begleitstoff 0.11 Gew.% Protein, bezogen auf den Polysaccharidgehalt.

0.3 g AW 115 gelöst in 1000 ml Lagerstättenwasser (Zusammensetzung siehe Beispiel 1) ergab bei einer Scherrate von 0.3 s⁻¹ und 40°C eine Viskosität von 51 mPas.

Beispiel 5

Herstellung eines Homopolysaccharides, Typ AW 116, mit dem Pilzstamm DSM 3891.

Ausgehend von einer Schrägargarkultur des Pilzstammes DSM 3891 wurde unter den gleichen Bedingungen, wie in Beispiel 4 beschrieben, das Homopolysaccharid AW 116 erzeugt. Es wurde jedoch als C-Quelle 50 g Maltose/1 Nährmedium eingesetzt. Die Kultivierung wurde nach 120 Stunden abgebrochen, nachdem ein pH-Wert von 3,6 erreicht und der Restgehalt an Maltose auf 0,1 Gew.% abgesunken war.
Nach Aufarbeitung gemäß dem Beispiel 4 wurden 4,0 g Homopolysaccharid erhalten mit unter 0,05 Gew.% Proteinen. Es ergaben 0,3 g AW 116, gelöst in 1000 ml Lagerstättenwasser der Zusammensetzung gemäß Beispiel 1, bei einer Scherrate von 0,3 s⁻¹ und 40°C eine Viskosität von 163.2 mPas.

Beispiel 6

Herstellung eines Homopolysaccharides, Typ AW 117, mit dem Pilzstamm DSM 3892.

Zwei Schrägagarkulturen des Stammes DSM 3892 wurden in je 3 ml 0,9 %iger Kochsalzlösung abgeschwemmt und damit 500 ml einer Nährsalzlösung, enthaltend 0.5 g Hefeextrakt, 0.5 g $K_2HPO_4$ • 3$H_2O$, 0.25 g $MgSO_4$ • 7 $H_2O$, 0.25 g Citronensäure • $H_2O$ und 5 g Saccharose beimpft. Unter Schütteln auf einer Rotationsschüttelmaschine bei 100 Upm wurde die Suspensionskultur bei 25°C 72 Stunden bei pH 5,6 gezüchtet. Mit dieser Vorkultur wurde ein 15 l-Bioreaktor, ausgerüstet mit einem Leitrohr mit Propellerrührer, beimpft, der 10 l Nährmedium folgender Zusammensetzung enthielt:

$K_2HPO_4$ • 3 $H_2O$          10.0 g
$MgSO_4$ • 7 $H_2O$          5.0 g

9

| Hefeextrakt | 5.0 g |
| Asparagin | 12.0 g |
| Glucose | 330.0 g |

Die Züchtung wurde 48 Stunden bei 25°C unter Rühren mit 600 Upm und einer Belüftung von 1.0 Liter steriler Luft pro Liter Nährmedium und Minute bei einem Anfangs-pH-Wert von 5.6 durchgeführt. Nach Beendigung der Kultivierung war der pH-Wert auf 3,2 abgefallen und der Rest-Glucose-Gehalt betrug 0.15 Gew.%.

Das Kulturmedium wurde mit 8 l deionisiertem Wasser versetzt, auf 85°C erhitzt und über eine Druckfiltration die Zellmasse abgetrennt. Die klare Kulturlösung wurde über eine Ultrafiltration mit einer Ausschlußgrenze von 100.000 Dalton auf einen Homopolysaccharid-Gehalt von 10 g/l aufkonzentriert und gefriergetrocknet. Es wurden 90 g Homopolysaccharid AW 117 gewonnen.

Nach Hydrolyse des Produktes in 6 N $H_2SO_4$ bei 90°C unter $N_2$-Atmosphäre wurde dünnschichtchromatographisch und mittels Hochdruckflüssigkeits-Chromatographie nur Glucose als Monomerkomponente festgestellt.

Die Elementaranalyse ergab folgende Zusammensetzung:

Kohlenstoff:  44,32 %
Wasserstoff:  6,21 %
Stickstoff:  0,09 %.

Daraus errechnet sich ein Restgehalt an Protein von 0,56 %.

Es ergaben 0,3 g AW 117, gelöst in 1000 ml Lagerstättenwasser gemäß Beispiel 1, bei einer Scherrate von 0,3 $s^{-1}$ und 40°C eine Viskosität von 144,5 mPas.

Um die Eignung der nach dem Verfahren der Erfindung erzeugten PS zur Tertiärförderung von Erdöl zu prüfen, wurden die im Anspruch 1 genannten Pilzstämme in Submerskultur gezüchtet, druckfiltriert und durch anschließende Ultrafiltration von anorganischen Ionen, von Restglucose und von Proteinen weitgehend befreit mit einer Ausschlußgrenze von 100.000 Dalton. Diese "Stammlösung" wurde mit Formaldehyd als Bakterizid und als Stabilisator versetzt.

Es wurden die folgenden Bewertungskriterien mit diesen "Stammlösungen" geprüft:

Die Viskosität in Lagerstättenwasser der Zusammensetzung gemäß Beispiel 1, mit einer Gesamtsalinität von 190 g/l.

Die Polymerkonzentration wurde so eingestellt, daß für die technische Anwendung bei 40°C bis 60°C die Viskositäten 30 bis 40 mPas bei einem Schergefälle D = 0,3 $s^{-1}$ betrugen. Zur Charakterisierung wurden die Viskositäten bei D = 0,3 $s^{-1}$ und D - $3^{-1}$ herangezogen.

Die Daten in der folgenden Tabelle zeigen, daß 0,3 g/l der erzeugten Homopolysaccharid-Lösungen ausreichen. Es wird festgestellt, daß die spezifische Viskosität bei 60°C höher und die Abhängigkeit von der Temperatur geringer ist als bei den zum Vergleich herangezogenen Handelsprodukten.

Die Tabelle zeigt, daß bei z.B. 40°C 1 g/l Flocon® 4800 gegenüber nur 0,3 g/l des erfindungsgemäß erhältlichen PS, Typ AW 114, aufzuwenden sind bei vergleichbaren Viskositäten.

Die Auswertung der Tabelle zeigt auch, daß dasselbe Handelsprodukt wegen seiner starken Abhängigkeit von der Temperatur bei T über 60°C nicht mehr zur Anwendung kommen kann. Dagegen weist das Homopolysaccharid nach dem Verfahren der Erfindung eine wenig temperaturabhängige Viskosität auf.

| Produkt +) | T = 25°C | Viskosität $\eta$ [mPa.s] | | Schergefälle D [$s^{-1}$] | Konzentration [g/l] |
|---|---|---|---|---|---|
| | | 40°C | 60°C | | |
| AW 114 | 74 30 | 53 26 | 44 22 | 0,3 3.0 | 0,3 |
| AW 117 | 48 24 | 39 20 | 27 16 | 0,3 3,0 | 0,3 |
| Actigum® | 20 12 | 13 9 | 8 7 | 0,3 3,0 | 0.3 |
| Actigum | 80 38 | 50 30 | 32 22 | 0,3 3,0 | 0,5 |
| Flocon® 4800 | - - | 42 34 | 18 14 | 0,3 3,0 | 1,0 |

+) Die Lösungen der Produkte sind unter jeweils gleichen Bedingungen geschert.

Das Handelsprodukt "Actigum" benötigt zur Einstellung der Viskosität von 30 bis 40 mPa.s bei D = 0,3 $s^{-1}$ und bei T = 60°C eine Menge von 0,5 g/l. Dagegen erfordert das nach dem Verfahren der Erfindung hergestellte Produkt, Typ AW 114, nur den Einsatz von 0,3 g/l.

Filtrationsverhalten:

Als technische Regel sollen die Lösungen der Homopolysaccharide durch Filter mit einem Porendurchmesser $D_p$ = 1,2 $\mu$m ohne Verstopfung frei fließen. Es werden 200 ml der Lösungen der Homopolysaccharide durch ein Filter von 47 mm Durchmesser bei einem Druck von 0,4 bar filtriert. Das Verhältnis der Durchflußrate des ersten Viertels zu der des vierten Viertels stelle das sogenannte "Milliporefilterratio" (MPFR) dar. Gut filtrierbare Lösungen weisen einen MPFR-Wert unter 1,6 auf. Dieses Filtrationsverhalten kann durch Scherung der "Stammlösungen" noch verbessert werden.

In der folgenden Tabelle sind die Daten der Viskositäten und der Filtration der nach dem Verfahren der Erfindung erzeugten PS mit den Pilzstämmen nach Anspruch 1 aufgeführt.

Es entsprechen die Typenbezeichnungen
AW: 106, 110, 114, 115, 116, 117 den Pilzstämmen:
DSM: 3889, 3887, 3888, 3890, 3891, 3892.
Es bedeuten: $\eta$ 0,3; $\eta$ 3.0 : Viskositäten bei Scherraten von 0,3 bzw 3,0 $s^{-1}$
MPFR = Milliporefilterratio
$D_p$ = Porendurchmesser
Konz. = Konzentration in g/l.
Als "Lösungsmittel" wurde Lagerstättenwasser gemäß dem Beispiel 1 verwendet.
Die "Scherbedingungen" sind:
7000 Upm x 3 min in einer "Ultraturrax-Umlaufapparatur" der Firma Janke & Kunkel, Staufen, DE.
Es bedeuten: - = ungeschert,
+ = geschert.
Alle Lösungen sind mit Membranfiltern mit 3 $\mu$m bzw. 5 $\mu$m filtriert. Die Viskositätsangaben nach der Filtration sind bei T = 25°C in mPa.s gemessen.

| Homopoly-saccharid | AW 106 | | AW 110 | | AW 114 | |
|---|---|---|---|---|---|---|
| | − | + | − | + | − | + |
| $\eta$ 0,3 | 46 | 57 | 31 | 118 | | 74 |
| $\eta$ 3,0 | 18 | 26 | 18 | 32 | | 30 |
| MPFR | 1,4 | 1,3 | 1,2 | 1,0 | | 1,0 |
| Dp [$\mu$m] | | 1,2 | 3 | | 1,2 | |
| Konz. [g/l] | | 0,3 | 0,3 | | 0,3 | |

| Homopoly-saccharid | AW 115 | | AW 116 | | AW 117 | |
|---|---|---|---|---|---|---|
| | − | + | − | + | − | + |
| $\eta$ 0,3 | 121 | 73 | 181 | 79 | 133 | 48 |
| $\eta$ 3,0 | 30 | 30 | 38 | 34 | 29 | 24 |
| MPFR | 1,5 | 1,8 | 1,8 | 1,6 | 1,16 | 1,0 |
| Dp [$\mu$m] | | 1,2 | 1,2 | | 1,2 | |
| Konz. [g/l] | | 0,4 | 0,3 | | 0,3 | |

Die Tabelle zeigt, daß die Typen AW 114 und AW 117 bereits ohne Scherung sehr gut filtrierbar sind. Durch die Scherung wird die Viskosität bevorzugt im niedrigen Bereich des Schergefälles, D = 0,3 s$^{-1}$ abgebaut. Es wird dadurch die Viskositätskurve abgeflacht, und die Viskosität kann durch diese Maßnahme eingestellt werden.

Langzeitstabilität:

Mit für die Praxis gescherten und filtrierten Lösungen wurde die Langzeitstabilität der Viskosität bei T = 40°C, 60°C, 85°C mit verschiedenen, mit 200 oder 1000 ppm Formaldehyd stabilisierten, Lösungen mit und ohne Ausschluß von Sauerstoff ($O_2$) geprüft.

Der Typ AW 114 ist beispielsweise bei 60°C nach einer Zeit von 1 Jahr und länger stabil. Dieses Ergebnis wird auch beispielsweise für den Type AW 117 bestätigt. Diese beiden Lösungen der Homopolysaccharide waren mit Fomaldehyd stabilisert, jedoch nicht frei von Sauerstoff.

Ein für die praktische Anwendung wichtiger Kennwert ist die Filtrationszeit für die erfindungsgemäß erhältlichen PS. In der folgenden Tabelle sind die Filtrationszeiten in min bei Einsatz von Membranfiltern mit 1,2 $\mu$m und mit 3 $\mu$m Porendurchmesser bei einem Filterdurchmesser von 47 mm aufgeführt. Zur Bestimmung der Filtrationseigenschaft werden 0,3 g/l PS in Lagerstättenwasser gemäß Beispiel 1 aufgelöst und bei 1 bar druckfiltriert über den Membranfiltern. Es wird die Zeit in min ermittelt, die für die Filtration von 200 ml erforderlich ist.

Es zeigen sich unterschiedliche Filtrationszeiten. Es zeigen native Lösungen der Typen AW 114 und AW 117 nach Ultrafiltration ohne eine Scherbehandlung gute Filtrationseigenschaften. Die Produkte AW 106, AW 110. AW 116, benötigen für gute Filtrationseigenschaften eine Scherbebehandlung von 5 sec. im Waring Blendor, um eine technisch brauchbare Filtrationszeit zu erzielen.

Der Typ AW 115 benötigt eine längere Filtrationszeit. Sofern es für die technische Anwendung auf eine kürzere Filtrationszeit ankommt, wird eine längere Scherzeit erforderlich. Die bevorzugten Typen der nach dem Verfahren der Erfindung hergestellten Homopolysaccharide gestatten Produkte mit den für die technische Anwendung erforderlichen Filtrationszeiten herzustellen.

Tabelle der Filtrationszeiten bei 2 unterschiedlichen Porendurchmessern der Menbranfilter in min.

| Typ | 3 $\mu$m [min.] | 1,2 $\mu$m [min.] |
|---|---|---|
| AW 106* | 0.5 | 18.0 |
| AW 110* | 3.0 | 12.0 |
| AW 114 | 0.8 | 4.0 |
| AW 115 | 10 | 14 |
| AW 116* | 1.6 | 1.6 |
| AW 117 | 2.5 | 4.5 |

* Scherbehandlung 5 sec. im Waring Blendor

Die nach dem Verfahren der Erfindung unter Verwendung von Mikroorganismen in Form von Pilzstämmen nach Anspruch 1 erzeugten PS bieten den Vorteil guter Wasserlöslichkeit. Diese Produkte sind daher und aufgrund ihrer rheologischen Eigenschaften in der Technik vielseitig anwendbar in Form von Lösungen, außerdem aber auch als geformte Massen oder in Form von Filmen und Fäden.

Weitere Vorteile ergeben sich aus der Temperaturstabilität, der Langzeitstabilität und der Scherstabilität der Lösungen, sowie aus deren einstellbarer Filtrierbarkeit.

**Patentansprüche**

1. Die Pilzstämme DSM 3887, DSM 3888, DSM 3890, DSM 3891, DSM 3892.

2. Verfahren zur extrazellulären Herstellung von Homopolysacchariden mit hoher spezifischer Viskosoität, dadurch gekennzeichnet, daß Mikroorganismen in Form von mindestens einem der Pilzstämme DSM 3887, DSM 3888, DSM 3890, DSM 3891, DSM 3892 in einem Nährmedium unter Belüftung und Bewegung bei einer Temperatur im Bereich von 15°C bis 40°C gezüchtet werden, danach die Kultursuspension im Falle ausgereifter Pilzstämme erhitzt, danach die Kulturlösung von der Zellmasse abgetrennt und das gebildete wasserlösliche Homopolysaccharid mit praktisch nur Glucose als Monomergrundkomponente daraus in üblicher Weise isoliert wird.

3. Verfahren nach Anspruch 2, dadurch gekennzeichnet, daß als Kohlenstoff-Quelle Stärke, Hemisellulose, Glucose oder Saccharose eingesetzt werden in einer solchen Konzentration, daß die Kultursuspension in dem 3-Phasen-System fest/flüssig/Gas noch mischbar ist.

4. Verfahren nach Anspruch 2 oder 3, dadurch gekennzeichnet, daß bei Pilzstämmen, deren 1, 3- oder 1,6-Glucanase oder beide positiv sind, eine C-Quelle einer solchen Konzentration eingesetzt wird, daß bei Abbruch der Kultivierung eine Restkonzentration der C-Quelle von etwa 0,1 Gew.%, bezogen auf die gesamte Lösung, besteht.

5. Verfahren nach einem der Ansprüche 2 bis 4, dadurch gekennzeichnet, daß die Pilzstämme an einer Trägersubstanz nach üblichen Methoden immobilisiert werden, und mit diesem Immobilisat die Bildung der Homopolysaccharide semikontinuierlich oder kontinuierlich durchgeführt wird.

**6.** Homopolysaccharid, erhältlich nach dem Verfahren der Patentansprüche 2 bis 5, mit einem durchschnittlichen Molekulargewicht von mindestens $8 \times 10^6$.

**7.** Verwendung des Homopolysaccharide nach Anspruch 6 als Dispergier- und Emulgiermittel, als Verdikkungsmittel, als Wasserrückhaltemittel und für die Verbesserung der Haftung von Stoffen an festen Oberflächen.

## Claims

**1.** The fungal strains DSM 3887, DSM 3888, DSM 3890, DSM 3891 and DSM 3892.

**2.** A process for the extracellular preparation of a homopolysaccharide having a high specific viscosity, wherein microorganisms in the form of one or more of the fungal strains DSM 3887, DSM 3888, DSM 3890, DSM 3891 and DSM 3892 are cultivated in a nutrient medium with aeration and agitation at from 15 to 40°C, the culture suspension is then heated if the fungal strains have matured, the culture solution is then separated from the cell mass, and the resulting water-soluble homopolysaccharide is isolated therefrom in a conventional manner, with virtually only glucose as a basic monomer component.

**3.** A process as claimed in claim 2, wherein starch, cellulose, hemicellulose, glucose or sucrose is used as the carbon source, in a concentration such that the culture suspension in the three-phase solid/liquid/gas system is still miscible.

**4.** A process as claimed in claim 2 or 3, wherein, in the case of fungal strains whose 1,3- or 1,6-glucanase is positive or whose 1,3- and 1,6-glucanase are both positive, a carbon source is used in a concentration such that the residual concentration of the carbon source is about 0.1% by weight, based on the whole solution, after termination of the cultivation.

**5.** A process as claimed in claim 2 or 3 or 4, wherein the fungal strains are immobilized on a substrate by conventional methods, and formation of the homopolysaccharide is carried out semicontinuously or continuously using this immobilized material.

**6.** A homopolysaccharide obtainable by the process of claim 2 or 3 or 4 or 5, having an average molecular weight of not less than $8 \times 10^6$.

**7.** Use of a homopolysaccharide as claimed in claim 6 as a dispersant, emulsifier, thickener and water retention agent and for improving the adhesion of substances to solid surfaces.

## Revendications

**1.** Les souches de champignons DSM 3887, DSM 3888, DSM 3890, DSM 3891, DSM 3892.

**2.** Procédé de préparation extracellulaire d'homopolysaccharides de viscosité spécifique élevée, caractérisé par le fait que l'on cultive, dans un bouillon de culture aéré et maintenu en mouvement, à des températures comprises entre 15 degrés C et 40 dégrés C, des microorganismes sous forme d'au moins une des souches de champignons DSM 3887, 3888, 3890, 3891, 3892, on chauffe ensuite la suspension de culture une fois les souches de champignons mûries, puis on sépare la solution de culture de la masse cellulaire et on isole de celle-ci de manière usuelle, l'homopolysaccharide formé, soluble dans l'eau, ne contenant pratiquement que du glucose comme composant monomère de base.

**3.** Procédé selon la revendication 2, caractérisé par le fait que l'on utilise, comme source de carbone, de l'amidon, de l'hémicellulose, du glucose ou du saccharose, en concentration telle que la suspension de culture est encore miscible dans le système à 3 phases : solide/liquide/gaz.

**4.** Procédé selon l'une des revendications 2 ou 3, caractérisé par le fait que pour des souches de champignons dont les glucanases 1,3 ou 1,6, ou les deux, sont positives, on utilise une source de carbone d'une concentration telle qu'un arrêtant la culture,il subsiste une concentration résiduelle de la source de carbone d'environ 0,1 % en poids, rapporté à la totalité de la solution.

5. Procédé selon l'une des revendications 2, 3 et 4, caractérisé par le fait que les souches de champignons sont immobilisées, selon des méthodes courantes, sur un matériau support et qu'avec la substance ainsi immobilisée on produit les homopolysaccharides en régime semi-continu ou continu.

6. Homopolysaccharide obtenu par le procédé des revendications 2 à 5, d'un poids moléculaire moyen d'au moins $8 \times 10^6$.

7. Utilisation de l'homopolysaccharide selon la revendication 6 comme dispersant et émulsifiant, comme épaississant, comme agent retenant l'eau et agent améliorant l'adhérence de substances sur des surfaces solides.